# FASCICULE DE BREVET EUROPEEN

(11) **EP 2 260 871 B1**
(45) Date de publication et mention de la délivrance du brevet: **15.05.2013**
(21) Numéro de dépôt: 10182089.2
(22) Date de dépôt: 29.03.2005
(51) Int. Cl.: A61K 47/48, A61P 29/00, B82Y 5/00

(54) **Complexes d'inclusions comprenant du piroxicam, une cyclodextrine et l'arginine**
Einschlusskomplexe enthaltend Piroxicam, ein Cyclodextrin und Arginin
Inclusion complexes comprising piroxicam, a cyclodextrin and arginine

(30) Priorité: 01.04.2004 FR 0403450; 21.10.2004 FR 0411201
(43) Date de publication de la demande: 15.12.2010
(62) Demande divisionnaire de: 05744579.3
(73) Titulaire: PIERRE FABRE MEDICAMENT, 92100 Boulogne-Billancourt (FR)
(72) Inventeur: Freiss, Bernard, 81100 Castres (FR); Marciacq, Florence, 81600 Brens (FR); Lochard, Hubert, 81600 Brens (FR)
(74) Mandataire: Regimbeau

(56) Documents cités:
- WO-A1-02/089851
- FR-A1- 2 830 760
- HEES VAN T ET AL: "APPLICATION OF SUPERCRITICAL CARBON DIOXIDE FOR THE PREPARATION OF DRUG-CYCLODEXTRIN INCLUSION COMPOUNDS", JOURNAL OF INCLUSION PHENOMENA AND MACROCYCLIC CHEMISTRY, KLUWER, DORDRECHT, NL, vol. 44, 2002, pages 271-274, XP008040794, ISSN: 1388-3127
- MURA P ET AL: "TERNARY SYSTEMS OF NAPROXEN WITH HYDROXYPROPYL-BETA-CYCLODEXTRIN AND AMINOACIDS", INTERNATIONAL JOURNAL OF PHARMACEUTICS, ELSEVIER BV, NL, vol. 260, no. 2, 24 juillet 2003 (2003-07-24), pages 293-302, XP008062550, ISSN: 0378-5173, DOI: DOI:10.1016/S0378-5173(03)00265-5
- HEES VAN T ET AL: "INCLUSION OF PIROXICAM INTO BETA-CYCLODEXTRIN BY MEANS OF SUPERCRITICAL CARBON DIOXIDE: THERMAL, SPECTROSCOPIC AND PHYSICOCHEMICALS STUDIES", JOURNAL DE PHARMACIE DE BELGIQUE, MASSON, PARIS, FR, vol. 55, no. 1, janvier 2000 (2000-01), pages 30-31, XP001019795, ISSN: 0047-2166
- HEES VAN T ET AL: "APPLICATATION OF SUPERCRITICAL CARBON DIOXIDE FOR THE PREPARATION OF A PIROXICAM-BETA-CYCLODEXTRIN INCLUSION COMPOUND", PHARMACEUTICAL RESEARCH, KLUWER ACADEMIC PUBLISHERS, NEW YORK, NY, US, vol. 16, no. 12, 1 décembre 1999 (1999-12-01), pages 1864-1870, XP001020308, ISSN: 0724-8741, DOI: DOI:10.1023/A:1018955410414

## Description

La présente invention concerne un procédé de préparation de composés d'inclusion solubles par la technologie des fluides denses sous pression, en particulier celle du CO₂.

De nombreuses substances actives, notamment d'intérêt dans le domaine pharmaceutique, présentent une très faible solubilité ou sont insolubles dans l'eau et par voie de conséquence dans les liquides biologiques. Ceci implique une faible biodisponibilité de ces substances actives et une forte augmentation des doses administrées aux patients pour atteindre l'objectif thérapeutique fixé et donc un accroissement des possibles effets secondaires liés aux traitements médicaux.

Les propriétés pharmacocinétiques d'une substance donnée dépendent entre autres de l'affinité de sa surface de contact pour le solvant considéré, à savoir l'eau dans le cas du domaine pharmaceutique. L'augmentation de la surface spécifique des poudres permet d'améliorer leur vitesse de dissolution. Or la biodisponibilité de principes actifs peut être considérablement augmentée si leur vitesse de dissolution est améliorée. Aussi, la formation de complexes moléculaires composés d'une ou plusieurs substances actives et d'une ou plusieurs molécule hôtes judicieusement choisi(es) pour sa (leur) forte solubilité dans les liquides biologiques peut donc permettre d'accroître la dissolution de la ou des substances actives dans les liquides biologiques.

Dans le domaine pharmaceutique, il existe un certain nombre de brevets et publications relatifs à la formation de complexes à base de cyclodextrine, en présence d'un agent d'interaction avec le complexe. Néanmoins, la plupart des documents ne proposent pas de procédés industriels, mais plutôt l'étude de l'amélioration de la solubilité d'un complexe substance active/cyclodextrine par le biais de l'agent d'interaction avec le complexe. Il s'agit en effet d'une énumération d'agents d'interaction avec le complexe testés pour un même principe actif, et des résultats analytiques observés. Par ailleurs, il existe peu de documents proposant une complexation en milieu supercritique.

Les documents décrivant des procédés de complexation avec une cyclodextrine par fluide supercritique sont les suivants :
Dans le but de fixer des molécules volatiles par inclusion, Kamihira M. et al (J. of Fermentation and Bioengineering, Vol. 69, N°6, 350-353, 1990**)** décrivent un procédé d'extraction de composés aromatiques volatiles et de piégeage par inclusion dans les cyclodextrines. Le géraniol et l'huile de moutarde sont ainsi extraits par un fluide sous pression, puis vaporisés en mode dynamique dans un second réacteur contenant des cyclodextrines. L'influence des différents paramètres est étudiée par mesure du taux d'inclusion des composés aromatiques dans les cyclodextrines. Toutefois, l'étape d'inclusion est réalisée en mode dynamique et non statique. Par ailleurs, l'application revendiquée par les auteurs est toute autre puisqu'il s'agit de fixation de molécules volatiles par inclusion. Enfin, ce procédé n'est pas mis en oeuvre avec des fluides supercritiques, mais avec des gaz sous pression.

Van Hees et al. (Pharmaceutical Research, Vol. 16, N° 12, 1999**)** décrivent dans leur publication un procédé d'inclusion de Piroxicam commercial dans les β -cyclodextrines par CO₂ supercritique. Le Piroxicam étant un anti-inflammatoire non-stéroïdien peu soluble dans l'eau, son inclusion dans les β-cyclodextrines doit permettre d'augmenter sa solubilité dans l'eau. Le procédé consiste à placer un mélange de Piroxicam et de β-cyclodextrines (ratio molaire 1/2,5) dans un autoclave pressurisé, laissé en mode statique. Après dépressurisation le mélange obtenu est broyé et homogénéisé. Le complexe est ensuite séché avant caractérisation par :
- DSC (Differential Scanning Calorimetry)
- Technique de la solubilité différentielle
- Méthodes spectroscopiques.

Ces analyses permettent de conclure quant au taux de complexation du Piroxicam avec la β-cyclodextrine. Il n'est pas mentionné l'importance d'un agent d'interaction avec le complexe sur la dissolution du complexe ainsi obtenu. Par ailleurs aucun agent de diffusion n'est utilisé dans l'étape de formation du complexe par CO₂ supercritique en mode statique.

La demande de brevet WO 03/043604 décrit un procédé de préparation de complexe moléculaire de substance active dans des molécules hôtes. Le procédé met en oeuvre une étape de diffusion moléculaire par mise en contact en mode statique, d'un fluide dense sous pression en présence éventuelle d'un agent de diffusion : l'eau.

Toutefois cette étape est suivie par une étape obligatoire de lavage par CO₂ supercritique. De plus aucun agent d'interaction avec le complexe n'est utilisé.

Différents documents font part de l'amélioration de la solubilité d'une substance active par addition d'agent d'interaction avec le complexe (Redenti, E. et al., J. of Pharmaceutical Sciences, Vol. 89, 1-8, 2000**)**. La solubilité de la substance active seule, de la substance active en présence de l'agent d'interaction avec le complexe, du complexe binaire substance active/cyclodextrine et enfin du complexe ternaire substance active/cyclodextrine/agent d'interaction avec le complexe sont étudiés. Toutefois aucun des procédés décrits n'utilise du CO₂ supercritique, ni en particulier une étape de diffusion moléculaire en mode statique en utilisant un agent de diffusion.

Ainsi, Buvári-Barcza et al. (J. of Inclusion Phenomena and Macrocyclic Chemistry, Vol. 42, 209-212, 2002**)** étudient la solubilité des complexes acide benzoïque/ β -cyclodextrine et benzène/β-cyclodextrine en présence d'acide acétique. La solubilité du complexe benzène/β-cyclodextrine est indépendante de la concentration en acide acétique alors que celle du complexe acide benzoïque/ β -cyclodextrine croît avec la concentration en acide acétique. L'interprétation des auteurs est la suivante : dans le complexe ternaire molécule /β-cyclodextrine/acide acétique, les liaisons hydrogène potentielles entre la molécule et la cavité intérieure de la cyclodextrine sont promoteurs d'autres interactions à l'extérieur de la cyclodextrine.

De même, Mura et al. (J. of Inclusion Phenomena and Macrocyclic Chemistry, Vol. 39, 131-138, 2001**)** mesurent la solubilité de l'éconazole en présence de cyclodextrines (α-, β-, γ-, hydroxypropyl-β-cyclodextrines) et d'hydroxyacides (acides tartarique, citrique, gluconique, malique et lactique). Les complexes ternaires sont préparés par mélange physique ou broyage des 3 composés, co-évaporation ou lyophilisation d'une solution contenant les 3 composés. La formation de complexe ternaire est suivie par DSC. Seul la lyophilisation permet d'obtenir un profil DSC ne présentant plus le pic de fusion de l'éconazole.

Les auteurs concluent à l'effet synergique observé dans le complexe ternaire, puisque les solubilités observées sont jusqu'à 20 fois plus élevées que celle d'un complexe binaire éconazole/cylodextrine.

Les mêmes auteurs (Int. J. of Pharmaceutics, Vol.260, 293-302, 2003**)** ont également étudié les complexes ternaires du type naproxen/hydroxypropyl-β-cyclodextrine/acide aminé. Les complexes cités sont préparés soit par co-broyage, soit par co-évaporation d'une solution eau/éthanol contenant les 3 composés.

Piel et al. , (J. of Pharmaceutical Sciences, Vol. 86-4, 475-480, 1997**)** présentent une étude de solubilité d'un complexe nimesulide/L-Lysine/β-ou γ-cyclodextrine obtenu par spray-drying ou évaporation. La solubilité du complexe ternaire est, selon le pH de la solution, jusqu'à 3600 fois plus importante que le nimesulide seul. Là encore, les auteurs parlent d'un effet synergique de la cyclodextrine et de la L-Lysine.

Fenivesy et al. (Proceedings of the 7th international cyclodextrins symposium, 414-418, 1994**)** s'intéressent à la complexation des substances actives terfénadine, dompéridone et astémizole avec l'hydroxypropyl-β-cyclodextrine en présence d'hydroxyacides.

Deux brevets (EP 0 991 407 et EP 1 018 340) décrivent la préparation de complexes ternaires substances actives/agent d'interaction avec le complexe/cyclodextrine. Les procédés mis en oeuvre sont le malaxage (kneading), le séchage par pulvérisation (spray-drying), l'évaporation ou la lyophilisation. Le procédé consiste soit à préparer le complexe du sel de la substance active, soit à mettre en contact simultanément les 3 composés au cours du procédé.

Un brevet (EP0153998 A2) déposé par Chiesi et al. décrit la préparation de complexes de piroxicam et de béta-cyclodextrine en présence notamment d'une solution ammoniacale. Toutefois, le procédé utilisé n'est pas mis en oeuvre avec du CO₂ supercritique.

Les deux seuls documents traitant de la préparation de complexe par CO₂ supercritique en présence d'agent d'interaction avec le complexe sont les suivants :
En suivant la même méthode que celle décrite précédemment (Pharmaceutical Research, Vol. 16, N° 12, 1999**),** Van Hees et al. (Journal of inclusion Phenomena and Macrocyclic Chemistry, N° 44, p 271-274, 2002**)** décrivent l'utilisation d'un agent d'interaction avec le complexe, la L-lysine ou le trométamol, dans la préparation d'un complexe Piroxicam/β-cyclodextrine par CO₂ supercritique.
L'utilisation de L-lysine ou de trométamol leur permet à la fois d'augmenter le taux d'inclusion du piroxicam dans la béta-cyclodextrine et d'améliorer la dissolution du complexe formé.

La caractérisation s'effectue par :
- DSC (Differential Scanning Calorimetry)
- Technique de la solubilité différentielle
- Cinétique de dissolution en milieu tamponné

Toutefois, aucun agent de diffusion n'est utilisé au cours de cette étape. V. Barillaro et al. (Proceeding of the 6th International Symposium on Supercritical Fluids, Versailles, p 1897-1902, 2003**)** se sont davantage focalisés sur l'amélioration que pouvait apporter l'ajout d'un agent acide d'interaction avec le complexe pour augmenter le taux d'inclusion du Miconazole dans des cyclodextrines. Différents agents d'interaction avec le complexe (acide malique, maléique, fumarique, citrique) ainsi que différentes cyclodextrines (béta-cyclodextrine, HP- béta-cyclodextrine, γ-cyclodextrine, HP- γ-cyclodextrine) ont été utilisés.

Toutefois, l'étape d'inclusion est réalisée en mode dynamique et non statique.

Dans les deux documents citées ci-dessus, il est donc important de noter que la complexation par fluides supercritiques est effectuée sur le mélange ternaire substance active/agent d'interaction avec le complexe/cyclodextrine et que par ailleurs l'agent d'interaction avec le complexe n'est pas l'arginine.

La molécule de piroxicam, représentée ci-dessous, possède une fonction énol et un cycle pyridine pouvant être salifiés ou non selon la valeur de pH du milieu de dissolution. 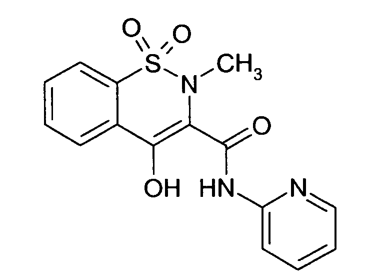 Wiseman et al. (Arzneim.- Forsch./Drug Res. 26 (7) 1976, 1300 - 1303 ) ont déterminé dans un mélange dioxane : eau 2:1 (v/v) la valeur de pKa de la fonction énol (pKa ∼ 2) et du cycle pyridine (pKa ∼ 6.3).

Selon la valeur de pH du milieu de dissolution, la molécule de piroxicam (PX) existera donc sous différentes formes, à savoir :
pH<2
   - *le cycle pyridine est protoné en ion pyridinium (NH⁺)*
   - la fonction énol n'est pas ionisée (-OH)
2 pH < 7
   - le cycle pyridine est protoné (NH⁺)
   - la fonction énol est ionisée en énolate (O⁻).
   C'est la structure zwittérionique du piroxicam. En terme de conformation moléculaire la structure zwittérionique est plane. Cette planéité résulte de liaisons hydrogène intramoléculaires entre l'anion énolate et le groupe amide d'une part puis la fonction carbonyle et le cation pyridinium d'autre part.
pH>7
   - la fonction énol est ionisée (O⁻)
   - le cycle pyridine n'est pas protoné (N).
   Les inventeurs ont découvert de façon surprenante que la séparation des étapes de complexation et d'ajout de l'agent d'interaction avec le complexe permet d'améliorer sensiblement les propriétés physico-chimiques du complexe ainsi obtenu. De plus, ils se sont également aperçus que l'utilisation de l'arginine comme agent d'interaction avec le complexe lorsque la substance active est le piroxicam permettait d'obtenir les complexes ayant les propriétés les plus intéressantes. L'objectif est d'améliorer la dissolution *in vivo* du piroxicam dans l'eau, et ce, en incluant le piroxicam dans un support poreux soluble puis en modifiant les propriétés physico-chimiques du système ainsi formé.

La présente inventiondécrit un procédé de préparation d'un composé d'inclusion soluble comprenant une ou plusieurs substances actives peu soluble dans un milieu aqueux incluses dans une ou plusieurs molécules hôtes, **caractérisé en ce qu**'il comprend les étapes successives suivantes:
a. mise en contact d'une ou plusieurs substances actives avec une ou plusieurs molécules hôtes,
b. mise en oeuvre d'une étape de diffusion moléculaire par mise en contact, en mode statique, d'un fluide dense sous pression avec le mélange obtenu à l'étape (a) en présence d'un ou plusieurs agents de diffusion,
c. récupération du complexe moléculaire substance active - molécule hôte ainsi formé,
d. mise en oeuvre d'une étape qui consiste à ajouter et mélanger un agent d'interaction avec le complexe au complexe moléculaire substance active - molécule hôte
e. récupération du composé d'inclusion soluble ainsi formé.
Dans le procédé selon la présente invention, il n'y a pas d'étape de lavage par CO₂ supercritique entre les étapes (c) et (d).
Dans un autre mode de réalisation avantageux, l'étape (e) est suivie par une étape (f) de séchage du composé d'interaction soluble, avantageusement entre 60°C et 80°C et de façon avantageuse pendant une nuit.

Le procédé selon la présente invention est ainsi constitué par l'enchaînement de deux phases qui sont :
- la formation d'un complexe d'inclusion entre une substance active et une molécule hôte en milieu supercritique (étapes (a), (b) et (c))
- la « fixation » de manière non-covalente d'un agent d'interaction avec le complexe sur le complexe obtenu, afin notamment d'améliorer ses propriétés physico-chimiques (étapes (d) et (e)).

L'invention décrit donc un procédé en cinq étapes.
- Les trois premières étapes dites de maturation, consistent essentiellement en une phase de diffusion moléculaire en milieu dense sous pression, et notamment supercritique permettant d'inclure des substances actives dans des molécules hôtes, notamment des cyclodextrines. L'objectif recherché au cours de cette phase de diffusion est de former des complexes d'inclusion entre des substances actives et la molécule hôte.

Par « complexe d'inclusion ou complexe moléculaire » on entend au sens de la présente invention tout complexe associant de manière non-covalente la substance active et la molécule hôte. Avantageusement, il s'agit du complexe issu de l'étape (b) du procédé selon la présente invention.

Par « composé d'inclusion soluble » on entend au sens de la présente invention toute entité formée par l'association de l'agent d'interaction avec le complexe d'une part et du complexe moléculaire d'autre part. Avantageusement, il s'agit du produit final obtenu par le procédé selon la présente invention.

Par « agent d'interaction avec le complexe», on entend au sens de la présente invention

la L-arginine..

Par «fluide dense sous pression», on entend au sens de la présente invention tout fluide utilisé à une température ou une pression supérieure à leur valeur critique. Avantageusement il s'agit du CO₂ pur ou en mélange avec un solvant organique classiquement utilisé par l'homme du métier.

Par « substance active peu soluble dans un milieu aqueux », on entend au sens de la présente invention le piroxicam.

Par « molécule hôte », on entend au sens de la présente invention les cyclodextrines et leur mélange. De façon avantageuse, il s'agit de la β-cyclodextrine, de la méthyl-β-cyclodextrine, de la γ-cyclodextrine ou de l'hydroxypropyl-β-cyclodextrine. Avantageusement, il s'agit de la β -cyclodextrine.

Les cyclodextrines sont des molécules dites « cages » car elles comportent, au sein de leur structure, une cavité relativement rigide et hydrophobe qui leur permet d'emprisonner ou d'encapsuler d'autres molécules. Le phénomène de complexation est la résultante d'une multitude d'interactions (substrat/solvant, solvant/solvant et cyclodextrine/solvant) qui conduisent à l'état thermodynamique le plus stable :
(1) les interactions de Van der Waals ;
(2) les interactions hydrophobes ;
(3) les liaisons hydrogène ;
(4) la libération de molécules d'eau avec une énergie élevée lors de la substitution par la molécule invitée ;
(5) la libération de l'énergie de tension au sein de la molécule de cyclodextrine lors de la formation du complexe.

Le composé d'inclusion soluble consiste en l'association du piroxicam, d'une cyclodextrine et de l'arginine, avantageusement la L-arginine.

Par « agent de diffusion », on entend au sens de la présente invention un agent de diffusion choisi dans le groupe constitué par les alcools, les cétones, les éthers, les esters et l'eau avec ou sans agent surfactant et leurs mélanges. De façon encore plus avantageuse, il s'agit de l'eau.

Par « mode statique » on entend au sens de la présente invention une réaction ou un procédé dans lequel tous les réactifs sont mis simultanément en présence et où on laisse la réaction se dérouler. Par exemple, dans l'étape (b) de la présente invention, on met dans un autoclave la ou les substance(s) active(s), de l'eau et du CO₂ supercritique et on laisse réagir pendant plusieurs heures. La masse de produit n'évolue pas durant la réaction. A l'inverse, en mode dynamique, les réactifs sont apportés au fur et à mesure de l'évolution de la réaction ou de la production. Souvent dans le cadre d'un mode dynamique, il y a circulation d'un fluide ou agitation. La masse de produit évolue durant la production.

Lors de l'étape (a) la substance active et la molécule hôte sont introduites sous forme solide ou liquide dans un récipient dans lequel lors de l'étape (b) est injecté le fluide dense sous pression et l'agent de diffusion dans des proportions judicieusement choisies. Les conditions de pression et de température ainsi que la durée du traitement sont définies, par toute méthode appropriée, en fonction de la nature de la ou des substances actives et du ou des molécules hôtes.

De façon avantageuse, l'étape (b) de diffusion moléculaire du procédé selon la présente invention est réalisée sous agitation.

L'agent de diffusion peut être ajouté en continu ou en discontinu dans une quantité comprise entre 1 et 50% en masse, de préférence entre 10 et 25% en masse.

Le ratio molaire substance active/molécule hôte/agent d'interaction avec le complexe pourra être choisi de manière à assurer la meilleure inclusion de la substance active au sein de la molécule hôte. Ainsi avantageusement le ratio molaire substance active/molécule hôte est compris entre 1/1 et 1/10, avantageusement entre 1/1 et 1/5, de façon avantageuse entre 1/2 et 1/2,5, de façon encore plus avantageuse 1/2,5. De même avantageusement le ratio molaire substance active/agent d'interaction avec le complexe est compris entre 1/1 et 1/3, avantageusement 1/1, plus avantageusement 1/1,2.

Le temps nécessaire à la diffusion moléculaire de l'étape (b) est déterminé par toute méthode appropriée. Cette étape (b) peut être réitérée autant de fois que souhaitée pour obtenir une vitesse de dissolution satisfaisante. Avantageusement, l'étape (b) dure entre environ 1 et 16 heures.

Les conditions de pression et de température de l'étape (b) sont choisies de façon à favoriser la diffusion moléculaire. Avantageusement la pression du fluide supercritique est comprise entre 0,5 MPa et 50 MPa et la température entre 0 et 200°C.

Avantageusement l'étape (b) du procédé selon la présente invention est mise en oeuvre dans un réacteur fermé, en particulier un autoclave.

Le procédé peut être mis en oeuvre en batch ou en continu. De façon avantageuse le procédé selon la présente invention est réalisé en batch.

Avantageusement, l'étape (b) du procédé selon la présente invention est mis en oeuvre dans un réacteur fermé éventuellement agité, alimenté par le fluide dense et la solution de substance active le cas échant, en continu.
- Les deux dernières étapes ((d) et (e)) consistent à ajouter et mélanger un agent d'interaction avec le complexe au complexe substance active - molécule hôte.

Cet agent d'interaction avec le complexe interagit selon deux hypothèses plausibles : interactions fortes avec la substance active incluse dans la molécule hôte lors des étapes précédente, et/ou interactions fortes avec le complexe précédemment formé.
Ceci permet d'améliorer principalement les propriétés de dissolution du complexe dans les liquides biologiques, et notamment l'eau et/éventuellement d'augmenter le taux d'inclusion de la substance active dans la molécule hôte.
L'amélioration des propriétés physico-chimiques, notamment en terme de dissolution du système formé peut avoir pour origine
- Une interaction non covalente de l'agent d'interaction avec le complexe avec la substance active, la molécule hôte ou les deux (complexation, salification...).
- Une variation locale du pH du milieu de dissolution
- L'obtention d'un système présentant un eutectique
- Une modification de l'interface entre le système et son milieu de dissolution (effet surfactant, changement granulométrie)
Avantageusement, l'étape (d) du procédé est mise en oeuvre en milieu semi-solide, le complexe n'étant pas dissous dans un milieu aqueux avant l'ajout de l'agent d'interaction avec le complexe. Cet agent va donc simplement humidifier le complexe ou former une pâte avec le complexe. Avantageusement, l'étape (d) est réalisée sous agitation.

La présente invention concerne un composé d'inclusion soluble comprenant du piroxicam peu soluble dans un milieu aqueux inclus dans une cyclodextrine et l'arginine comme agent d'interaction avec le complexe, susceptible d'être obtenu par le procédé selon la présente invention. Avantageusement le taux d'inclusion de la substance active dans le composé d'inclusion soluble selon la présente invention est supérieur à 95% calculé par analyse DSC telle que décrite ci-après, avantageusement supérieur à 98 %, avantageusement proche de 99 %.

Avantageusement, la solubilité du principe actif lorsqu'il se trouve sous la forme du complexe selon la présente invention est supérieure à 2,5 g/l, avantageusement au moins égale à 3 g/l. Avantageusement, le taux de solubilisation de la substance active contenue dans une solution à 4 g/l dans l'eau mesurée à 38°C après entre 5 et 120 minutes d'agitation est supérieure à 63 %, avantageusement au moins égale à 75 %. La substance active est le piroxicam et la solubilité et le taux de solubilisation sont mesurés à pH = 6,3.

La molécule hôte est la cyclodextrine, la substance active est le piroxicam.

Avantageusement, le piroxicam est présent dans le complexe selon la présente invention sous une structure zwittérionique.

Dans le cadre de la présente invention, on entend par « taux de solubilisation » le pourcentage de piroxicam solubilisé après 5 à 120 minutes d'agitation à 37°C d'un mélange d'eau et de piroxicam. On utilisera usuellement pour mesurer ce taux un mélange à 4g/l de piroxicam dans l'eau. Cette solubilisation peut être mesurée par un test de solubilisation tel qu'indiqué ci-après (test de solubilisation du piroxicam).

La présente invention concerne en outre un complexe comprenant du piroxicam, une cyclodextrine et de l'arginine **caractérisé en ce que** le taux de solubilisation du piroxicam contenue dans une solution à 4 g/l dans l'eau, mesuré à 37°C après entre 5 et 120 minutes d'agitation est supérieure à 90%, avantageusement supérieure à 95%, de façon avantageuse égale à 99%.

Par « cyclodextrines », on entend au sens de la présente invention les cyclodextrines, les cyclodextrines modifiées et leurs mélanges. De façon avantageuse, il s'agit de la β-cyclodextrine, de la méthyle-β-cyclodextrine, de la γ -cyclodextrine ou de l'hydroxypropyl-β-cyclodextrine. Avantageusement, il s'agit de la β-cyclodextrine.

Avantageusement, le complexe piroxicam-cyclodextrine-arginine selon la présente invention présente une bonne cristallinité, de façon avantageuse, ce complexe présente moins de 40 % en poids de phase amorphe, de façon encore plus avantageuse moins de 30 % en poids de phase amorphe, avantageusement une quantité de phase amorphe inférieure ou égale à 20 % en poids.

Avantageusement, le taux d'inclusion du piroxicam dans le complexe piroxicam-cyclodextrine-arginine selon la présente invention mesurée par l'analyse calorimétrique différentielle telle que décrite ci-après est supérieur à 98 %, avantageusement supérieur à 99 %, de façon avantageuse proche de 100 %. Avantageusement, le piroxicam est présent dans le complexe selon la présente invention sous une structure zwittérionique.

Les complexes piroxicam, cyclodextrine et arginine selon la présente invention sont susceptibles d'être obtenus par le procédé tel que décrit ci-dessus. Toutefois, ils sont également susceptibles d'être obtenus par le procédé ci-dessous.

Ainsi, un procédé de préparation d'un complexe piroxicam-cyclodextrine-arginine selon la présente invention comprend les étapes successives suivantes :
(1) mise en contact du piroxicam avec une cyclodextrine et l'arginine,
(2) mise en oeuvre d'une étape de diffusion moléculaire par mise en contact, en mode statique, d'un fluide dense sous pression avec le mélange obtenu à l'étape (1) en présence d'un ou plusieurs agents de diffusion,
(3) récupération du complexe piroxicam-cyclodextrine-arginine ainsi formé.
   Ce procédé permet dans le cas des complexes piroxicam-cyclodextrine-arginine d'obtenir des complexes ayant des propriétés particulières.

L'étape (2) de diffusion moléculaire en mode statique appelée étape de maturation, consiste essentiellement en une phase de diffusion moléculaire en milieu dense sous pression, et notamment supercritique, permettant d'inclure le piroxicam dans les cyclodextrines. L'objectif recherché au cours de cette phase de diffusion est de former des complexes d'inclusion entre le piroxicam, la cyclodextrine et l'arginine. Le complexe ainsi formé associe de manière non-covalente le piroxicam, la cyclodextrine et l'arginine.

L'arginine qui sert d'agent d'interaction interagit selon deux hypothèses plausibles : interactions fortes avec le piroxicam inclus dans la cyclodextrine et/ou interactions fortes avec le complexe formé.

La présence de l'arginine permet d'améliorer principalement les propriétés de dissolution du complexe dans les liquides biologiques, et notamment l'eau.

L'amélioration des propriétés physico-chimiques, notamment en terme de dissolution du système formé peut avoir pour origine
- Une interaction non covalente de l'arginine avec le piroxicam, la cyclodextrine ou les deux (complexation, salification...).
- Une variation locale du pH du milieu de dissolution
- L'obtention d'un système présentant un eutectique
- Une modification de l'interface entre le système et son milieu de dissolution (effet surfactant, changement granulométrie).

Par «fluide dense sous pression», on entend au sens de la présente invention tout fluide utilisé à une température ou une pression supérieure à leur valeur critique. Avantageusement il s'agit du CO₂ pur ou en mélange avec un solvant organique classiquement utilisé par l'homme du métier.

Par « agent de diffusion », on entend au sens de la présente invention un agent de diffusion choisi dans le groupe constitué par les alcools, les cétones, les éthers, les esters et l'eau avec ou sans agent surfactant et leurs mélanges. De façon encore plus avantageuse, il s'agit de l'eau.

Par « mode statique » on entend au sens de la présente invention une réaction ou un procédé dans lequel tous les réactifs sont mis simultanément en présence et où on laisse la réaction se dérouler. Par exemple, dans l'étape (2) de la présente invention, on met dans un autoclave le piroxicam, l'eau, l'arginine et du CO₂ supercritique et on laisse réagir pendant plusieurs heures. La masse de produit n'évolue pas durant la réaction. A l'inverse, en mode dynamique, les réactifs sont apportés au fur et à mesure de l'évolution de la réaction ou de la production. Souvent dans le cadre d'un mode dynamique, il y a circulation d'un fluide. La masse de produit évolue durant la production.

De façon avantageuse, l'étape (2) de diffusion moléculaire du procédé selon la présente invention est réalisée sous agitation.

Dans un mode de réalisation particulier de l'invention, lors de l'étape (1) le piroxicam, l'arginine et les cyclodextrines sont introduits sous forme solide ou liquide dans un récipient dans lequel est injecté le fluide dense sous pression et l'agent de diffusion dans des proportions judicieusement choisies. Les conditions de pression et de température ainsi que la durée du traitement sont définies, par toute méthode appropriée.

Le ratio molaire piroxicam/cyclodextrine/arginine pourra être choisi de manière à assurer la meilleure inclusion du piroxicam au sein des cyclodextrines. Ainsi avantageusement le ratio molaire piroxicam/cyclodextrine est compris entre 1/1 et 1/10, avantageusement entre 1/1 et 1/5, de façon avantageuse entre 1/2 et 1/2,5, de façon encore plus avantageuse 1/2,5. De même avantageusement le ratio molaire piroxicam/arginine est compris entre 1/1 et 1/3, avantageusement 1/1, plus avantageusement 1/1,2.

L'agent de diffusion peut être ajouté en continu ou en discontinu dans une quantité comprise entre 1 et 50% en masse par rapport à la masse totale du mélange, avantageusement entre 20 et 25% en masse par rapport à la masse totale du mélange.

Le temps nécessaire à la diffusion moléculaire de l'étape (2) est déterminé par toute méthode appropriée. Cette étape (2) peut être réitérée autant de fois que souhaitée pour obtenir une vitesse de dissolution satisfaisante. Avantageusement, l'étape (2) dure entre environ 2 et 16 heures, avantageusement 1 heure.

Les conditions de pression et de température de l'étape (2) sont choisies de façon à favoriser la diffusion moléculaire. Avantageusement la pression du fluide supercritique est comprise entre 5 MPa et 40 MPa, avantageusement 15MPa et la température entre 0 et 120°C, avantageusement 100°C.

Avantageusement l'étape (2) du procédé selon la présente invention est mise en oeuvre dans un réacteur fermé, en particulier un autoclave.

Le procédé peut être mis en oeuvre en batch ou en continu. De façon avantageuse le procédé selon la présente invention est réalisé en batch.

Avantageusement, l'étape (2) du procédé est mis en oeuvre dans un réacteur fermé éventuellement agité, alimenté par le fluide dense et le piroxicam et le cas échant, en continu.

La mise en oeuvre de l'étape de diffusion moléculaire en milieu dense sous pression en présence d'un agent de diffusion permet une forte interaction des particules de piroxicam avec les cyclodextrines, ce qui favorise la dissolution en milieu aqueux.

La présente invention concerne en outre une composition pharmaceutique comprenant un complexe piroxicam-cyclodextrine-arginine selon la présente invention, et éventuellement un excipient pharmaceutiquement acceptable.

La présente invention concerne également un complexe piroxicam-cyclodextrine-arginine selon la présente invention ou une composition pharmaceutique selon la présente invention en tant que médicament, avantageusement ayant une action anti-inflammatoire et avantageusement destiné à traiter les des rhumatismes inflammatoires, la polyarthrite, l'arthrose, les tendinites ou les affections post-traumatiques de l'appareil locomoteur.

Les exemples suivants sont donnés à titre indicatif.

Les différents exemples proposés ont été réalisés avec du piroxicam comme substance active de la béta cyclodextrine comme molécule hôte et l'eau comme agent de diffusion. L'ammoniaque ou l'arginine ont été utilisées comme agent d'interaction avec le complexe.

### Description des figures

La figure 1 représente le spectre RMN du complexe obtenu selon l'exemple 7.
La figure 2 représente le spectre ROESY-NOE intramoléculaire du complexe obtenu selon l'exemple 7.
La figure 3 représente le profil TG-DGT (analyse thermogravimétrique) de la β -cyclodextrine en utilisant le procédé suivant : augmentation de température de 5°C par minute jusqu'à 450°C puis diminution de la température de 10°C par minute jusqu'à 80°C.
La figure 4 représente le profil DSC de la β-cyclodextrine en utilisant la méthode suivante : un équilibrage à 0°C suivie d'une augmentation de la température de 5°C par minute jusqu'240 °C.
La figure 5 représente le profil de l'analyse thermogravimétrique de la L-arginine en utilisant le même procédé que celui de la figure 3.
La figure 6 représente le profil DSC de la L-arginine en utilisant le même procédé que celui de la figure 4.
La figure 7 représente le profil DSC du piroxicam en utilisant le même procédé que celui de la figure 6.
La figure 8 représente le profil DSC d'un échantillon de piroxicam fondu à 260°C puis refroidit à 0°C en utilisant le procédé d'équilibrage à 205°C, de diminution de la température de 20°C par minute jusqu'à 0°C et d'augmentation de la température de 5°C par minute jusqu'à 220°C.
La figure 9 représente le profil DSC du complexe obtenu selon l'exemple 7 en utilisant le procédé d'équilibrage à 0°C, d'augmentation de la température de 5°C par minute jusqu'à 240°C puis de diminution de la température de 5°C par minute jusqu'à 80°C.
La figure 10 représente le profil TG-DGT du complexe obtenu selon l'exemple 7 en utilisant le procécé d'augmentation de la température de 5°C par minute jusqu'à 450°C puis de diminution de la température de 10°C par minute jusqu'à 80°C.

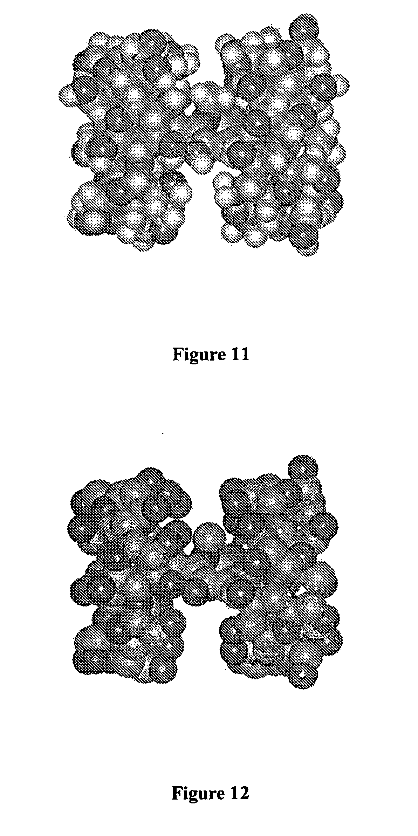

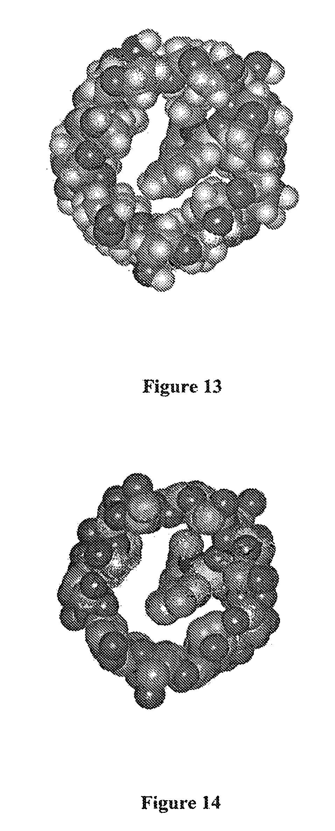

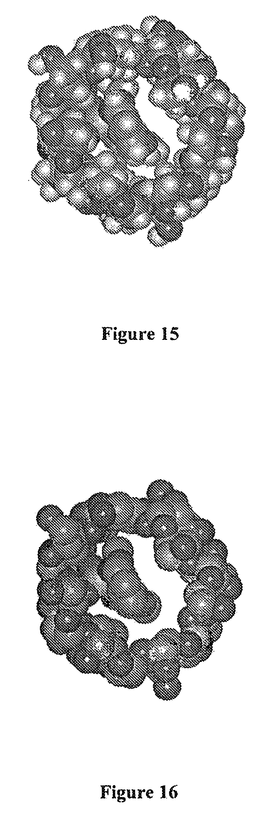

Les figures 11 à 16 représentent la modélisation moléculaire de la structure minimisée du complexe d'inclusion piroxicam- β-cyclodextrine 1 : 2. En particulier la figure 11 représente la vue de profil avec les atomes d'hydrogène, la figure 12 représente la vue de profil sans les atomes d'hydrogène, la figure 13 représente la vue de la cavité côté cycle benzothiazine avec les atomes d'hydrogène, la figure 14 représente la vue de la cavité côté cycle benzothiazine sans les atomes d'hydrogène, la figure 15 représente la vue de la cavité côté cycle pyridine avec les atomes d'hydrogène et la figure 16 représente la vue de la cavité côté cycle pyridine sans les atomes d'hydrogène.
La figure 17 représente le diffractogramme rayons X du complexe obtenu selon l'exemple 7 et du complexe obtenu avec le procédé du brevet EP 0 153 998.

### Analyse :

Le taux d'inclusion de la substance active dans la molécule hôte est évalué par analyse thermique différentielle (DSC).

Les analyses DSC des complexes obtenus selon les exemples 1 à 6 ont été réalisées de la façon suivante :
On applique une rampe de température sous flux d'azote au produit à tester, à l'aide d'un appareil DSC 7 Perkin Elmer.

Le rendement de complexation est évalué par mesure de la réduction (ou disparition) du pic thermique relatif à la fusion du principe actif « resté libre » sous forme cristalline.

L'analyse DSC du complexe obtenu selon l'exemple 7 a été réalisée de la façon suivante :
L'appareillage, DSC Q100 TA Instruments, a été calibré à l'aide du signal de fusion de l'indium sous un flux d'azote de 50mL.min⁻¹. L'échantillon est analysé en nacelle hermétique de 5°C.min⁻¹.

Les analyses thermogravimétriques sont réalisées à l'aide de l'appareillage TGA2950 HR TA Instruments afin de déterminer le domaine de stabilité thermique des échantillons. L'appareil est calibré à la température ambiante et à l'aide du point de Curie du nickel à 360,46°C. L'exactitude de la balance est vérifiée par l'analyse d'un échantillon d'oxalate de calcium.

L'analyse est réalisée sous un flux d'azote de 60 mL.min⁻¹ de 25°C à 450°C.

La quantité d'eau dans les échantillons est déterminée par coulométrie à l'aide du coulomètre Mettler KF DL37. L'appareillage est étalonné avec le STANDARD tartrate de sodium dihydrate (%H₂O=15,66 ± 0,05 %).

La diffraction des rayons X de poudres est réalisée avec l'appareillage et les conditions suivantes :
- diffractomètre Philips Xpert MPD, générateur Philips tension 40kV intensité 20mA
- anticathode de cuivre (Kalpha = 1.5418367 angstrom), filtre Ni
- fente entrée 1/8
- détecteur Xcelerator
- mode scan continu
- échantillon broyé en poudre sur plaquette
- domaine angulaire (°2θ) 4 à 100
- durée du scan : 80 secondes

L'analyse des phases est réalisée à l'aide du logiciel Visual CRYSTAL.

Les spectres de diffusion Raman sont obtenus avec l'appareillage et les conditions suivantes :
- Spectromètre Raman Labram HR 800 Jobin Yvon
- Température: 22° C
- Echantillons: poudre sur lamelle microscope
- Longueur d'onde excitatrice: 752 nm, puissance laser 10mW sur l'échantillon
- Résolution spectrale 2 cm-1, volume diffusant environ 1µm3 (réseau 600 traits)

Le spectre RMN proton est enregistré sur un spectromètre DPX AVANCE Bruker à la fréquence nominale de 400 MHz en utilisant une sonde inverse large bande équipée d'un accessoire de gradient de champ selon l'axe Z. Le spectromètre est préalablement calé sur la fréquence de résonance du deutérium du solvant de solubilisation, en l'occurence le diméthylsulfoxide-d₆ (Eurisotop, réf. D 310B, lot A 2731) Les déplacements chimiques sont donnés en p.p.m. par rapport au T.M.S. (tétraméthylsilane) utilisé comme standard interne.

Le spectre ROESY (**R**otating frame **O**verhauser **E**nhancement **S**pectroscopY) est obtenu en appliquant le microprogramme d'impulsions Bruker « *roesytp.2* ». L'enregistrement est réalisé en mode phasé avec 1024 incrémentations et 72 balayages par incrément soit une durée totale d'expérience de 53 heures par produit. L'acquisition est réalisée en sélectionnant une fenêtre spectrale de 6410,256 Hz, un délai de relaxation de 2 s et un temps de verrouillage de spin de 350 ms. Préalablement à l'enregistrement du spectre l'échantillon est correctement dégazé afin d'observer le maximum d'effet NOE intra et intermoléculaire.

Compte tenu de la faible solubilité des complexes dans l'eau et de la sensibilité du spectromètre RMN en notre possession nous avons préféré travailler dans le diméthylsulfoxide deutérié dans lequel il est possible d'atteindre une concentration de 2% (p/v) en complexe.

Modélisation moléculaire : L'optimisation du complexe d'inclusion piroxicam-β-cyclodextrine (1 :2) est réalisée avec le logiciel Hyperchem^{®}, version 6.02 (Hypercube, Gainsville, USA), implémenté sur un ordinateur personnel pentium III modèle HP Vectra.

La structure moléculaire du piroxicam utilisée pour les calculs de minimisation est extraite de la publication de Jon Bordner et coll. (Acta Cryst., 1984, C40, 989 - 990).

La structure moléculaire de la β-cyclodextrine provient de la Cambridge Crystallographic Database (M.R. Caira et al., J. Chem. Soc., Chem. Commun, 1994, 1061 - 1062).

La minimisation d'énergie de la géométrie et de la conformation du complexe est réalisée à l'aide du champ de force MM2.

La simulation des propriétés physico-chimiques (pKa, logD et solubilité) à partir de la molécule de piroxicam est réalisée avec le logiciel ACD/logD suite (ACD/labs software, Toronto, Canada).

Pour mesurer les propriétés de dissolution de la poudre, on dissout l'équivalent de 4g/l de piroxicam dans une solution aqueuse, à 37°C. Au bout de 15 min, on effectue un prélèvement, puis on mesure par HPLC la quantité de piroxicam dissoute, le résultat est exprimé en grammes de piroxicam dissous par litre d'eau.

Selon cette méthode la solubilité du piroxicam pur dans l'eau est inférieure à 0,2 g/l.

### TEST DE SOLUBILISATION DU PIROXICAM.

### Protocole opératoire :

Le dosage du Piroxicam dans la solution de dissolution est réalisé par HPLC :
Appareillage utilisé :
   Système HPLC WATERS :
      - Module de séparation 2695,
      - Détecteur UV 2487.
Conditions chromatographiques :
   Colonne : WATERS X-Terra MS C18 250x4,6 mm.
   *1.1.1.1.1.1 Phase mobile : Voie A*
   60 % Tampon pH=3.5 KH₂PO₄ 6,81 g/l ajusté au pH avec H₃PO₄ dilué R,
   40 % acétonitrile.
   Débit : 1 ml / min
   Longueur d'onde détecteur: 230 nm
   Sensibilité du détecteur : 2 AUFS
   Volume injecté : 20 µl
   Température du four : 40°C
   Temps d'analyse : 12 minutes
Préparation des solutions témoins:
   Solution témoin : SM : Introduire 50 mg de Piroxicam témoin dans une fiole de 100 ml. Dissoudre avec 20 ml de diméthylformamide et compléter au volume avec du méthanol. 1.
   Gamme :
      T1 : Dilution au 1/20^{ème} de T3 dans 40 acétonitrile / 60 eau.
      T2 : Dilution au 1/10^{ème} de T3 dans 40 acétonitrile / 60 eau.
      T3 : Dilution au 1/100^{ème} de SM dans 40 acétonitrile / 60 eau.
      T4 : Dilution au 1/50^{ème} de SM dans 40 acétonitrile / 60 eau.
      T5 : Dilution au 1/20^{ème} de SM dans 40 acétonitrile / 60 eau.

### Conditions opératoires des cinétiques de dissolution à 4 g/L:

### Réalisation de l'essai :

### Conditions opératoires :

Dans un erlen-meyer de 100 ml, introduire une prise d'essai équivalente à 200 mg de Piroxicam. Ajouter 50 ml d'eau. Mettre sous agitation magnétique à 400 tours par minutes, dans un bain thermostaté à 37°C +/- 2°C. Effectuer un prélèvement de 2 ml sous agitation magnétique à 5, 30, 60, 120 minutes. Filtrer les prélèvements sur filtre polypropylène 0,45µm Gelman GHP Acrodisc. La solution doit être limpide.

Diluer le prélèvement au 1/200^{eme} dans la phase mobile.

### Méthodologie, expression des résultats :

Injecter 20 µl de chaque solution témoin.

Effectuer une régression linéaire des surfaces des pics de Piroxicam par rapport aux concentrations. Le coefficient de corrélation doit être supérieur à 0,995.

Injecter 20 µl des solutions à examiner.

Mesurer l'aire du pic de Piroxicam dans chaque solution à examiner.

En déduire la concentration X en µg/ml d'après la droite de régression des témoins.

Calculer la concentration en µg par ml de Piroxicam solubilisé en multipliant par l'inverse de la dilution réalisée (i.e. : 200).

Le taux de solubilisation du piroxicam est calculé en divisant la concentration de piroxicam solubilisé par la concentration de piroxicam totale de la solution de départ.

### Exemple comparatif 1 : Résultats obtenus en utilisant le procédé selon la présente invention

8 grammes de piroxicam, 76 grammes de béta-cyclodextrine, 25,2 g d'eau sont mélangés et introduits dans un réacteur de deux litres. Le dioxyde de carbone est par la suite introduit dans le réacteur sous une pression de 200 bar et sous une température de 150°C. L'ensemble est maintenu dans ces conditions opératoires pendant une durée de deux heures.

Après l'étape dite de maturation, une partie de la poudre collectée (12 g) est mélangée à 2,11 g d'une solution ammoniacale à 28%, puis placée dans une étuve ventilée à 60°C, durant une nuit.

L'analyse DSC révèle un taux d'inclusion du piroxicam dans la cyclodextrine de 99%, la dissolution du piroxicam est de 3,019 g/l.

### Exemple comparatif 2 : Résultats obtenus après l'étape de maturation sans ajout d'agent d'interaction avec le complexe

4 grammes de piroxicam, 38 grammes de béta-cyclodextrine, 8,95 grammes d'eau sont mélangés et introduits dans un réacteur de deux litres. Le dioxyde de carbone est par la suite introduit dans le réacteur sous une pression de 150 bar et sous une température de 150°C. L'ensemble est maintenu dans ces conditions opératoires pendant une durée de deux heures.

Après détente du milieu une partie de la poudre collectée est placée dans une étuve ventilée à 60°C, durant une nuit. L'analyse DSC révèle un taux d'inclusion du piroxicam dans la cyclodextrine de 80 %, la dissolution du piroxicam est de 0,246 g/l.

### Exemple comparatif 3 : Résultats obtenus si l'agent d'interaction avec le complexe est présent au cours de l'étape de maturation (b)

2 g de piroxicam, 19 g de béta-cyclodextrine, 3,75 g d'eau et 1,5 g de solution ammoniacale à 28% sont mélangés et introduits dans un réacteur de deux litres. Le dioxyde de carbone est par la suite introduit dans le réacteur sous une pression de 200 bar et sous une température de 160°C. L'ensemble est maintenu dans ces conditions opératoires pendant une durée de deux heures.

Après détente du milieu, la poudre collectée est placée dans une étuve ventilée à 60°C, durant une nuit. L'analyse DSC révèle un taux d'inclusion du piroxicam dans la cyclodextrine de 50 %, la dissolution du piroxicam est de 1,075g/l.

### Exemple comparatif 4: Résultats obtenus si l'on tente de fixer l'agent d'interaction à la molécule hôte avant l'étape de maturation (b)

19 g de béta-cyclodextrine et 2,11 g de solution ammoniacale à 28% et 3,15 g d'eau purifié sont mélangés et placés dans une étuve ventilée à 60°C, durant une nuit. On ajoute ensuite 2 g de piroxicam et 6,57 g d'eau. Le mélange est ensuite introduit dans un réacteur de deux litres. Le dioxyde de carbone est par la suite introduit dans le réacteur sous une pression de 200 bar et sous une température de 160°C. L'ensemble est maintenu dans ces conditions opératoires pendant une durée de deux heures.

L'analyse DSC révèle un taux d'inclusion du piroxicam dans la cyclodextrine de 92 %, la dissolution du piroxicam est de 0,23 g/l.

### Exemple comparatif 5: Résultats obtenus si l'on tente de fixer l'agent d'interaction à la substance active avant l'étape de maturation (b)

2 g de piroxicam et 2 g de solution ammoniacale à 28% sont mélangés et placés dans une étuve ventilée à 60°C, durant une nuit. On ajoute ensuite 14,3 g de béta-cyclodextrine et 3,3 g d'eau. Le mélange est ensuite introduit dans un réacteur de deux litres. Le dioxyde de carbone est par la suite introduit dans le réacteur sous une pression de 150 bar et sous une température de 150°C. L'ensemble est maintenu dans ces conditions opératoires pendant une durée de deux heures.

L'analyse DSC révèle un taux d'inclusion du piroxicam dans la cyclodextrine de 56 %, la dissolution du piroxicam est de 1,370g/l.

### Exemple comparatif 6 : Résultats en utilisant le procédé selon la présente invention sur des lots de taille semi-industrielle : 12,5 kg

1,1 kg de piroxicam, 10,6 kg de béta-cyclodextrine, 1,3 kg d'eau sont mélangés et introduits dans un réacteur de 50 litres. Le dioxyde de carbone est par la suite introduit dans le réacteur sous une pression de 150 bar et sous une température de 100°C. L'ensemble est maintenu dans ces conditions opératoires pendant une durée de deux heures.

Une partie de la poudre est placée dans une étuve à 80°C pendant une nuit. L'analyse DSC révèle un taux d'inclusion du piroxicam dans la cyclodextrine de 89 %, la dissolution du piroxicam est de 1,937g/l.

La poudre collectée (12,6 kg) est mélangée à 2,6 kg d'une solution ammoniacale à 28%, puis placé dans une étuve ventilée à 60°C.

L'analyse DSC révèle un taux d'inclusion du piroxicam dans la cyclodextrine de 100 %, la dissolution du piroxicam est supérieure à 3 g/l.

### Résumé des résultats obtenus

Le tableau 1 ci-dessous rassemble les résultats des différents exemples 1 à 6 indiqués ci-dessus et permet de constater la valeur ajoutée du procédé selon la présente invention.

**Tableau 1 :**

| **Procédé / Matières** | **/** | **Ex.1** | **Ex. Comp. 2** | **Ex. Comp. 3** | **Ex. Comp. 4** | **Ex. Comp. 5** | **Ex. 6** |
|---|---|---|---|---|---|---|---|
| Matières engagées : | | | | | | | |
| • Piroxicam | X | X | X | X | X | | X |
| • Piroxicam préalablement mélangé avec l'agent d'interaction avec le complexe | | | | | | X | |
| • β-cyclodextrine | | X | X | X | | X | X |
| • β-cyclodextrine préalablement mélangé avec l'agent d'interaction avec le complexe | | | | | X | | |
| 1^{ère} phase : Complexation en milieu CO₂ supercritique | | X | X | | X | X | X |
| 2^{ème} phase : ajout de l'agent d'interaction avec le complexe | | X | | | | | X |
| 1 seule phase : Complexation avec l'agent d'interaction avec le complexe en milieu CO₂ supercritique | | | | X | | | |
| | | | | | | | |
| | | | | | | | |
| **Taux de complexation (DSC)** | **/** | **99%** | **80%** | **50%** | **92%** | **56%** | **100 %** |
| **Dissolution (g/l)** | **< 0,2** | **3,02** | **0,25** | **1,07** | **0,23** | **1,37** | **> 3** |

### Exemple 7 : complexe Piroxicam, β-cyclodextrine, arginine

43 gramme de piroxicam, 384 gramme de β-cyclodextrine et 25 gramme d'arginine sont introduits dans un réacteur, ainsi que 61 grammes d'agent de diffusion (eau). Le dioxyde de carbone est par la suite introduit dans le réacteur sous une pression de 15 MPa et sous une température de 100°C. L'ensemble est maintenu dans ces conditions opératoires pendant une durée d'une heure.

Sur les complexes obtenus, sont mesurés la cinétique de dissolution et le taux de solubilisation comme indiqué ci-dessus dans le « Test de solubilisation DU PIROXICAM ». Les résultats sont rassemblés dans le Tableau 2 ci-après.

| Temps (minutes) | Concentration en piroxicam (µg/ml) | Taux de solubilisation (%) |
|---|---|---|
| 5 | 3863 | 96,6 |
| 30 | 3854 | 96,4 |
| 60 | 3941 | 98,5 |
| 120 | 3962 | 99 |

### RMN:

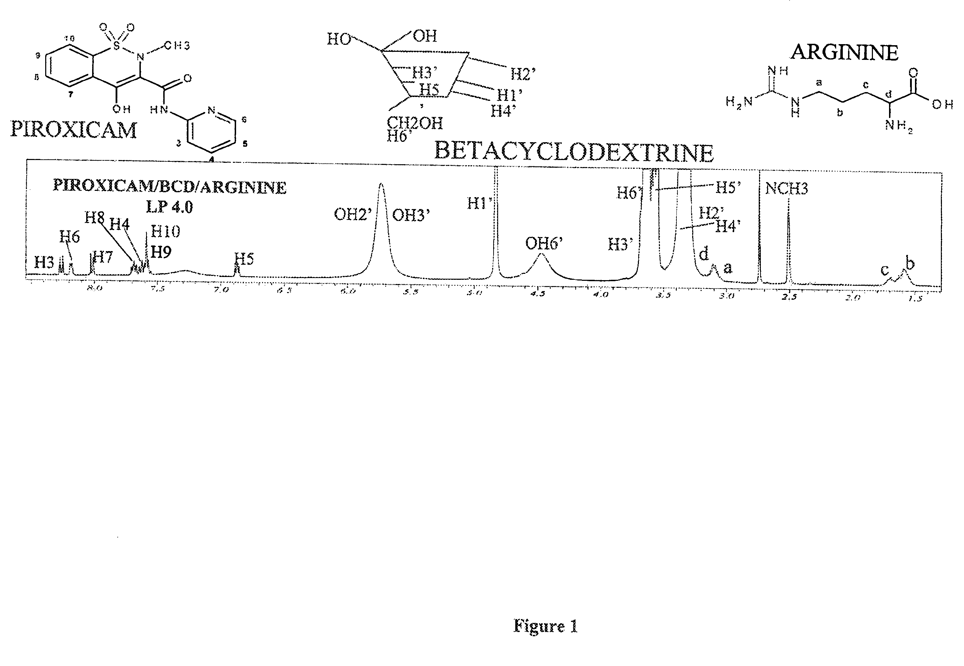

**Le spectre RMN ¹H** d'un complexe obtenu selon l'exemple 7 (figure 1) est en accord avec la composition chimique des de ce composé.

Le signal observé à 13,5 p.p.m. est attribué au proton pyridinium apportant une preuve supplémentaire de l'existence d'une structure zwittérionique du piroxicam dans le complexe obtenu selon l'exemple 7.

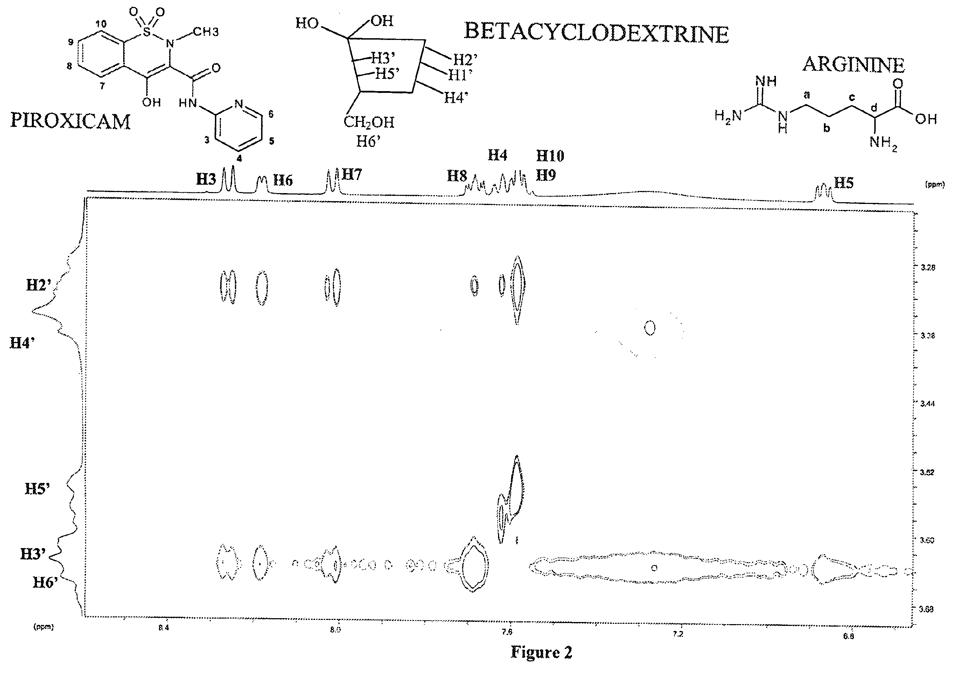

### Analyse du spectre ROESY (figure 2)

Cette étude qui permet d'étudier les transferts de magnétisation entre des protons proches dans l'espace va nous éclairer sur le mode d'inclusion du piroxicam dans la β -cyclodextrine et ainsi nous permettre de modéliser le complexe correspondant.

A partir de la carte ROESY nous distinguons les effets NOE intramoléculaires (entre des protons de la même molécule) des effets NOE intermoléculaires (entre des protons appartenant à des molécules différentes). Si les effets NOE intramoléculaires sont, bien entendu, instructifs en terme d'analyse conformationnelle nous ne citerons que les effets NOE intermoléculaires qui apportent la preuve d'une encapsulation du piroxicam dans la β-cyclodextrine. L'analyse du spectre ROESY enregistré sur un complexe obtenu selon l'exemple 7 (figures 2) montre clairement des pics de relaxation croisée entre H-9/10 du piroxicam et H-5' de la βCD puis entre H-7, H-6, H-3 du piroxicam et H-3' de la β -cyclodextrine. Ces résultats sont en accord avec un complexe d'inclusion 1 : 2, piroxicam : β-cyclodextrine.

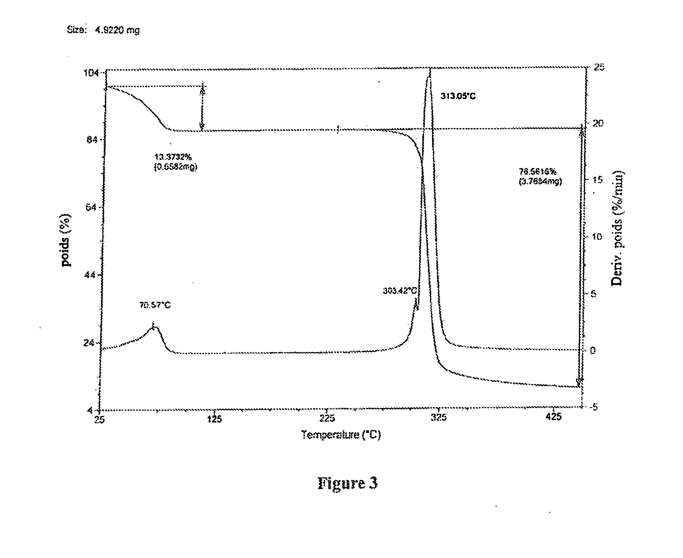

**Analyse thermique et dosage d'eau** : L'analyse thermogravimétrique de la β cyclodextrine (figure 3) met en évidence deux transitions à 70,6°C et 313°C. La première correspond à une perte de poids de 13,4% en accord avec la quantité d'eau déterminée par coulométrie (14,1%), la deuxième transition est attribuée à la décomposition thermique de la β cyclodextrine.

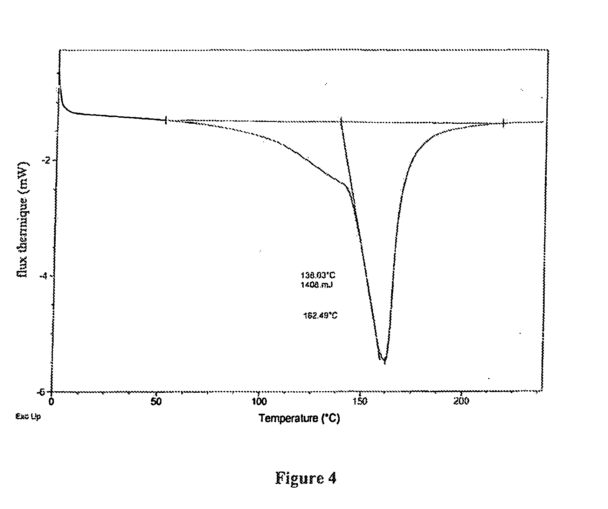

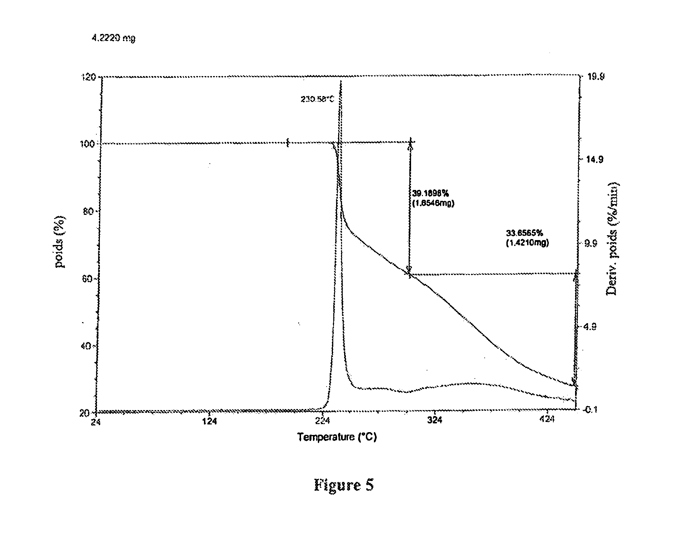

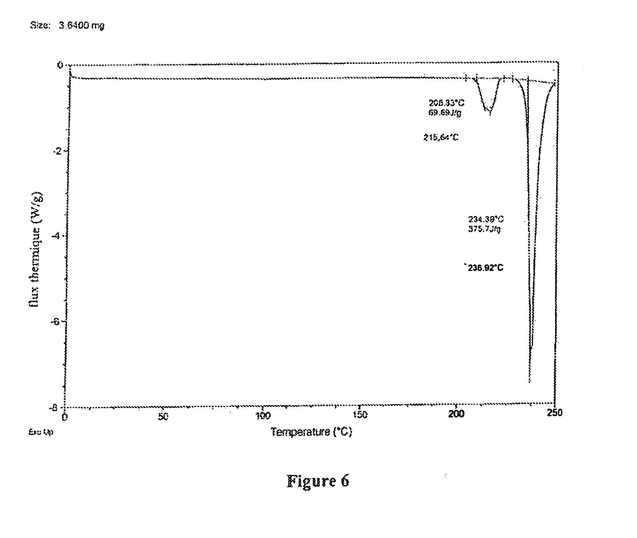

L'analyse DSC (figure 4) montre un large endotherme centré sur 162°C correspondant au phénomène de déshydratation. L'analyse thermogravimétrique de l'arginine (figure 5) montre que celle ci est stable jusqu'aux environs de 230°C avant de se décomposer. Deux endothermes sur le profil DSC sont observés (figure 6), le premier à 215,6°C (fusion ou transition de phases) le second à 234°C lié au phénomène de décomposition.

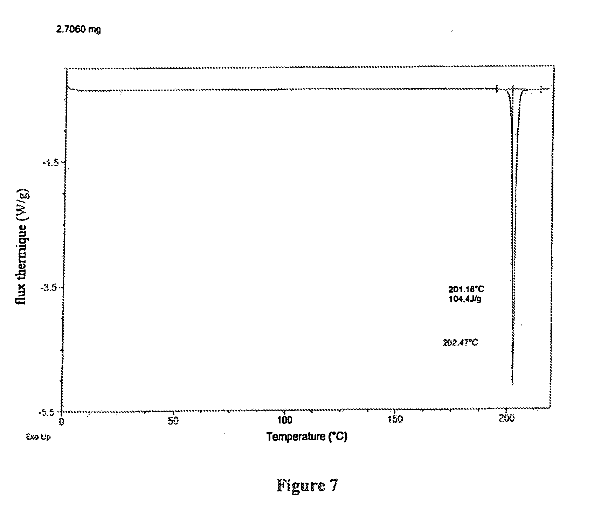

Le profil DSC du piroxicam montre que la fusion intervient à 201°C avec une enthalpie de 104 J.g⁻¹ (figure 7).

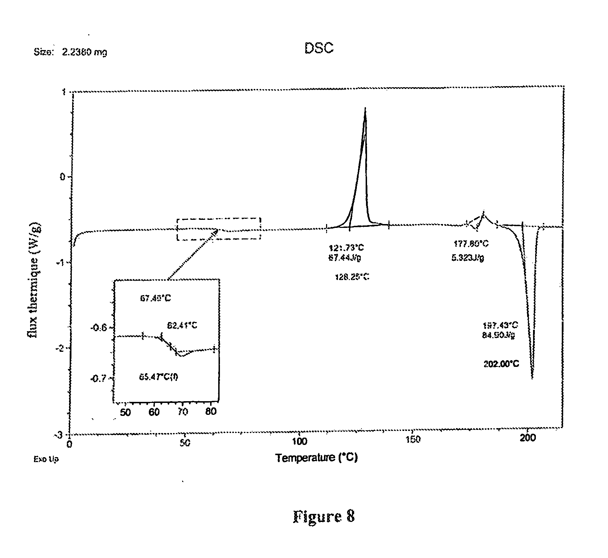

Des cycles chauffage refroidissement sont réalisés, dans lesquels un échantillon de piroxicam est fondu à 205°C puis refroidi à 0°C. Le profil DSC de cet échantillon (figure 8) révèle l'existence d'une transition vitreuse à 62°C caractéristique de l'état amorphe qui cristallise à 122°C. Un faible endotherme à 178°C suivi d'un exotherme puis d'un dernier endotherme à 197°C traduit l'existence de plusieurs formes cristallines du piroxicam (polymorphisme).

Les deux endothermes à 178°C et 197°C seraient attribués respectivement à la fusion des formes III et II alors que l'échantillon initial avant le traitement thermique correspondrait à la forme I (fusion à 201°C) (F. Vre 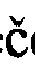 er , S. Sr 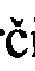 i 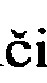 i and 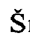 mid-Korbar, International Journal of Pharmaceutics 68 (1991) 35-41).

La teneur en eau du complexe obtenu selon l'exemple 7 est de l'ordre de 10% p/p, comme indiqué dans le tableau 3 suivant :

**Tableau 3 - résultat des dosages d'eau du complexe de l'exemple 7**

| complexe | teneur H₂O (TGA) | teneur H₂O (coulométrie) |
|---|---|---|
| obtenu selon Exemple 7 | 9,99% | 10,14 % |

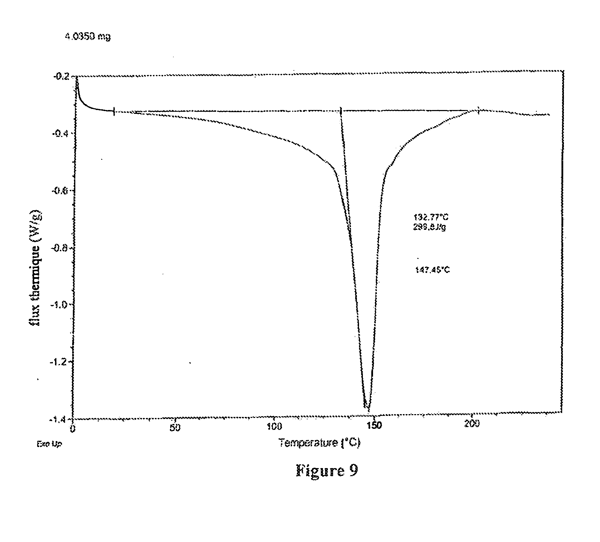

Le profil DSC du complexe obtenu selon l'exemple 7 (figure 9) ne montre pas les transitions à 198-200°C et à 218° suggérant une inclusion proche de 100 %.

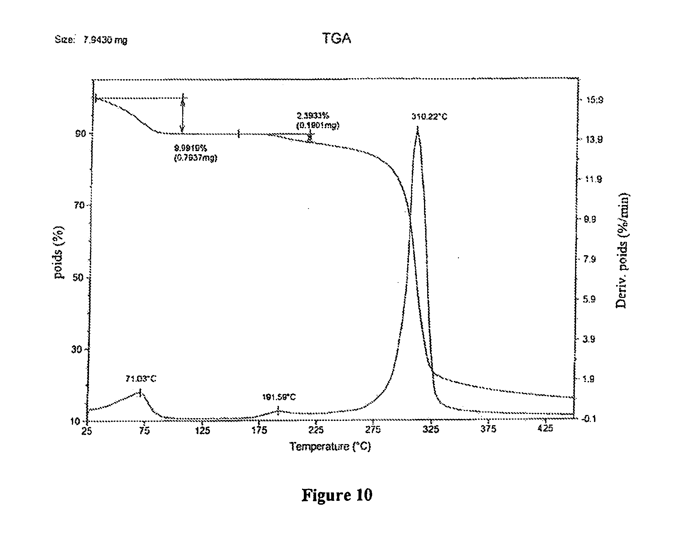

Le profil TG DTG du complexe obtenu selon l'exemple 7 (figure 10) montre une transition à 191°C. Nous attribuons cette transition à la décomposition de l'arginine. En effet, la première étape de décomposition de l'arginine correspond à une perte de poids de l'ordre de 39,2% et si nous tenons compte de la teneur en arginine dans le complexe, environ 6% p/p (détermination par RMN), cette perte de poids devrait-être de l'ordre de 2,35% p/p, en excellent accord avec celle observée sur les profils TG et DTG à 191°C (2,40%).

Modélisation moléculaire : La structure minimisée du complexe d'inclusion piroxicam: β-cyclodextrine (1 :2) est représentée aux figures 11 à 16. Cette structure optimisée tient compte des interactions spatiales observées par spectromètrie ROESY.

Comme il est indiqué dans la publication de G.M. Escandar (Analyst, 1999, 124, 587 - 591), la molécule de piroxicam est trop volumineuse (environ 6 x 13,7 Â) pour être entièrement encapsulée dans une cavité de β-cyclodextrine. Avec un complexe PX:(βCD)₂ la molécule de piroxicam est totalement incluse.

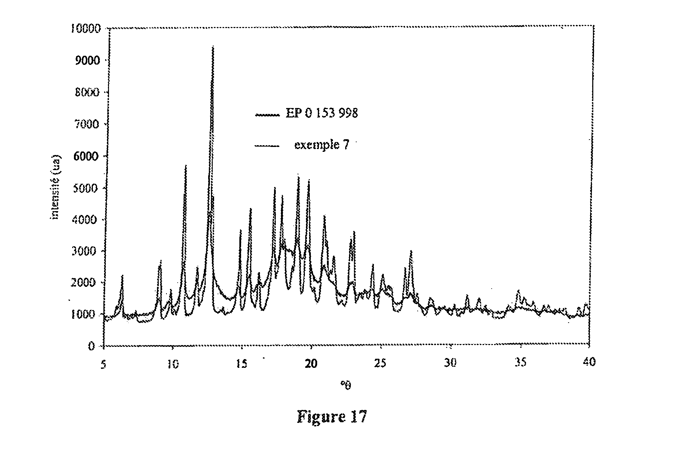

Simulation des propriétés physico-chimiques : variation de solubilité du piroxicam en fonction du pH du milieu de dissolution. La structure zwittérionique présente entre pH 2 et 6, la solubilité la plus faible. La valeur de logD évolue quant à elle en sens inverse. Dans l'intervalle de pH 2 - 6, la molécule est relativement hydrophobe ce qui représente une autre interaction favorable à l'encapsulation. Entre pH 6 et pH 7,5 le zwittérion est encore présent et la valeur de solubilité augmente sensiblement en fonction du pH. D'où l'intérêt de préparer des complexes dans cette zone de pH afin d'additionner les deux effets solubilisants qui sont l'encapsulation d'une part et la salification du piroxicam d'autre part. Les valeurs mesurées de pH sont indiquées ci-après : complexe selon l'exemple 7 à 13,9 mg/10 ml H₂O = pH 7,87 Diffractions aux rayons X du complexe obtenu selon l'exemple 7 et du complexe obtenu selon le procédé décrit dans EP 0 153 998 : le diffractogramme est représenté à la figure 17. Le diffractogramme de poudre du complexe selon l'exemple 7 montre des raies de diffraction intenses et très bien résolues apportant la preuve d'une meilleure cristallinité de cet échantillon par rapport à celui du complexe selon le procédé du brevet EP 0 153 998. Selon le logiciel Visual CRYSTAL, le complexe obtenu selon l'exemple 7 présente entre 16 et 20 % en poids de phase amorphe.

### Exemple 8 : complexe Piroxicam, β-cyclodextrine, arginine

400 grammes de piroxicam, 3832 grammes de β-cyclodextrine et 253 grammes d'arginine sont introduits dans un réacteur, ainsi que 613 grammes d'agent de diffusion (eau). Le dioxyde de carbone est par la suite introduit dans le réacteur sous une pression de 15 MPa et sous une température de 100°C. L'ensemble est maintenu dans ces conditions opératoires pendant une durée d'une heure.

Sur les complexes obtenus, sont mesurés la cinétique de dissolution et le taux de solubilisation comme indiqué ci-dessus dans le « Test de solubilisation DU PIROXICAM ». Les résultats sont rassemblés dans le Tableau 3 ci-après.

| Temps (minutes) | Concentration en piroxicam (µg/ml) | Taux de solubilisation (%) |
|---|---|---|
| 5 | 3953 | 98,8 |
| 30 | 3895 | 97.3 |
| 60 | 3952 | 98.8 |
| 120 | 4041* | 100 |

| | | |
|---|---|---|
| *Résultat en cohérence avec l'incertitude liée à la mesure. | | |

## Revendications

1. Complexe d'inclusion comprenant du piroxicam, une cyclodextrine et l'arginine **caractérisé en ce que** le taux de solubilisation du piroxicam contenue dans une solution à 4 g/l dans l'eau, mesuré à 37°C après entre 5 et 120 minutes d'agitation est supérieure à 90%, avantageusement supérieure à 95%, de façon avantageuse égale à 99%.

2. Complexe d'inclusion comprenant du piroxicam, une cyclodextrine et de l'arginine **caractérisé en ce qu'**il comprend moins de 40 % en poids de phase amorphe, avantageusement moins de 30 % en poids.

3. Complexe selon l'une quelconque des revendications 1 ou 2 **caractérisé en ce que** la cyclodextrine est une béta-cyclodextrine.

4. Procédé de préparation d'un complexe selon l'une quelconque des revendications 1 à 3 **caractérisé en ce qu'**il comprend les étapes successives suivantes :
(1) mise en contact du piroxicam avec une cyclodextrine et l'arginine,
(2) mise en oeuvre d'une étape de diffusion moléculaire par mise en contact, en mode statique, d'un fluide dense sous pression avec le mélange obtenue à l'étape (a) en présence d'un agent de diffusion choisi dans le groupe constitué par les alcools, les cétones, les éthers, les esters et l'eau avec ou sans agent surfactant et leurs mélanges,
(3) récupération du complexe piroxicam-cyclodextrine-arginine ainsi formé.

5. Procédé selon la revendication 4 **caractérisé en ce que** le fluide dense sous pression est le dioxyde de carbone.

6. Procédé selon l'une quelconque des revendications 4 ou 5 **caractérisé en ce que** l'agent de diffusion est l'eau.

7. Procédé selon l'une quelconque des revendications 4 à 6 **caractérisé en ce que** l'étape (2) de diffusion moléculaire est réalisée avec agitation.

8. Procédé selon l'une quelconque des revendications 4 à 7 **caractérisé en ce que** l'agent de diffusion est ajouté en continu ou en discontinu dans une quantité comprise entre 1 et 50% en masse par rapport à la masse totale du mélange, de préférence entre 10 et 25% en masse par rapport à la masse totale du mélange.

9. Composition pharmaceutique comprenant un complexe selon l'une quelconque des revendications 1 à 3, et éventuellement un excipient pharmaceutiquement acceptable.

10. Complexe selon l'une quelconque des revendications 1 à 3 ou composition pharmaceutique selon la revendication 9 en tant que médicament, avantageusement ayant une action anti-inflammatoire et avantageusement destiné à traiter les rhumatismes inflammatoires, la polyarthrite, l'arthrose, les tendinites ou les affections post-traumatiques de l'appareil locomoteur.

## Patentansprüche

1. Einschlusskomplex, welcher Piroxicam, ein Cyclodextrin und Arginin umfasst, **dadurch gekennzeichnet, dass** die Solubilisationsrate von Piroxicam, welches in einer Lösung mit 4 g/l in Wasser enthalten ist, gemessen bei 37 °C nach 5- bis 120-minütiger Agitation über 90%, vorteilhafterweise über 95 %, in vorteilhafter Weise bei 99 % liegt.

2. Einschlusskomplex, welcher Piroxicam, ein Cyclodextrin und Arginin umfasst, **dadurch gekennzeichnet, dass** er weniger als 40 Gew.-%, vorteilhafterweise weniger als 30 Gew.-%, amorphe Phase umfasst.

3. Komplex gemäß irgendeinem der Ansprüche 1 oder 2, **dadurch gekennzeichnet, dass** das Cyclodextrin ein Beta-Cyclodextrin ist.

4. Verfahren zur Herstellung eines Komplexes gemäß irgendeinem der Ansprüche 1 bis 3, **dadurch gekennzeichnet, dass** es die folgenden aufeinanderfolgenden Schritte umfasst:
(1) In-Kontakt-Bringen von Piroxicam mit einem Cyclodextrin und Arginin,
(2) Durchführen eines Schrittes molekularen Diffusion durch In-Kontakt-Bringen, im statischen Modus, eines unter Druck stehenden dichten Fluids mit der in Schritt (a) erhaltenen Mischung in Gegenwart eines Diffusionsmittels, welches aus der Gruppe bestehend aus Alkoholen, Ketonen, Ethern, Estern und Wasser mit oder ohne oberflächenaktiven Stoff und deren Mischungen ausgewählt ist,
(3) Gewinnen des auf diese Weise gebildeten Piroxicam-Cyclodextrin-Arginin-Komplexes.

5. Verfahren gemäß Anspruch 4, **dadurch gekennzeichnet, dass** das unter Druck stehende dichte Fluid Kohlendioxid ist.

6. Verfahren gemäß irgendeinem der Ansprüche 4 oder 5, **dadurch gekennzeichnet, dass** das Diffusionsmittel Wasser ist.

7. Verfahren gemäß irgendeinem der Ansprüche 4 bis 6, **dadurch gekennzeichnet, dass** der Schritt (2) der molekularen Diffusion mit Rühren bewirkt wird.

8. Verfahren gemäß irgendeinem der Ansprüche 4 bis 7, **dadurch gekennzeichnet, dass** das Diffusionsmittel kontinuierlich oder diskontinuierlich in einer Menge von 1 bis 50 Massen-%, bezogen auf die Gesamtmasse der Mischung, bevorzugt von 10 bis 25 Massen-%, bezogen auf die Gesamtmasse der Mischung, zugegeben wird.

9. Pharmazeutische Zusammensetzung, welche einen Komplex gemäß irgendeinem der Ansprüche 1 bis 3 und, gegebenenfalls, einen pharmazeutisch akzeptablen Exzipienten umfasst.

10. Komplex gemäß irgendeinem der Ansprüche 1 bis 3 oder pharmazeutische Zusammensetzung gemäß Anspruch 9 als Medikament, vorteilhafterweise mit entzündungshemmender Wirkung und vorteilhafterweise zur Behandlung entzündlicher Rheumaerkrankungen, von Polyarthritis, Arthrose, Sehnenentzündungen oder posttraumatischen Schädigungen des Bewegungsapparats.

## Claims

1. Inclusion complex comprising piroxicam, a cyclodextrin and arginine **characterized in that** the solubilization rate of the piroxicam contained in a 4 g/l solution in water, measured at 37°C after between 5 and 120 minutes of stirring, is greater than 90%, advantageously greater than 95%, advantageously equal to 99%.

2. Inclusion complex comprising piroxicam, a cyclodextrin and arginine **characterized in that** it comprises less than 40% by weight of amorphous phase, advantageously less than 30% by weight.

3. Complex according to either one of Claims 1 or 2 **characterized in that** the cyclodextrin is a β-cyclodextrin.

4. Process for the preparation of a complex according to any one of Claims 1 to 3 **characterized in that** it comprises the following successive steps:
(1) bringing piroxicam into contact with a cyclodextrin and arginine,
(2) carrying out a step of molecular diffusion by bringing a dense pressurized fluid into contact, in static mode, with the mixture obtained in step (a) in the presence of a diffusion agent selected from the group consisting of alcohols, ketones, ethers, esters and water with or without a surfactant and mixtures thereof,
(3) recovery of the piroxicam-cyclodextrin-arginine complex thus formed.

5. Process according to Claim 4 **characterized in that** the dense pressurized fluid is carbon dioxide.

6. Process according to either one of Claims 4 or 5 **characterized in that** the diffusion agent is water.

7. Process according to any one of Claims 4 to 6 **characterized in that** the molecular diffusion step (2) is carried out with stirring.

8. Process according to any one of Claims 4 to 7 **characterized in that** the diffusion agent is added continuously or discontinuously in an amount of between 1 and 50% by mass in relation to the total weight of the mixture, preferably between 10 and 25% by mass in relation to the total mass of the mixture.

9. Pharmaceutical composition comprising a complex according to any one of Claims 1 to 3, and optionally a pharmaceutically acceptable excipient.

10. Complex according to any one of Claims 1 to 3 or pharmaceutical composition according to Claim 9 as a medicament advantageously having an anti-inflammatory action and advantageously intended to treat inflammatory rheumatism, polyarthritis, arthrosis, tendinitis or post-traumatic conditions of the locomotor apparatus.
